# FASCICULE DE BREVET EUROPEEN

(11) **EP 0 568 695 B1**
(45) Date de publication et mention de la délivrance du brevet: **06.09.1995**
(21) Numéro de dépôt: 93901796.8
(22) Date de dépôt: 27.11.1992
(51) Int. Cl.: A61K 7/09

(54) **COMPOSITION COSMETIQUE POUR UNE PERMANENTE DES CHEVEUX CONTENANT EN TANT QUE REDUCTEUR DU BROMHYDRATE DE CYSTEINE ET/OU DU BROMHYDRATE DE MERCAPTO-2 ETHYLAMINE**
Kosmetische Zusammensetzung zur Haarbehandlung
COSMETIC COMPOSITION FOR HAIR PERMING CONTAINING CYSTEIN BROMHYDRATE AND/OR 2-MERCAPTO ETHYLAMINE BROMHYDRATE AS THE REDUCING AGENT

(30) Priorité: 27.11.1991 FR 9114643
(43) Date de publication de la demande: 10.11.1993
(73) Titulaire: L'OREAL, 75008 Paris (FR)
(72) Inventeur: LEROY, Frédéric, F-92210 Saint-Cloud (FR); MALLE, Gérard, F-77580 Villiers-sur-Morin (FR); BURANDE, Agnès, F-77270 Vileparisis (FR); DUVAULT, Yolanda, F-93320 Les Pavillons-sous-Bois (FR)
(74) Mandataire: Stalla-Bourdillon, Bernard
(86) Numéro de dépôt international: FR9201108
(87) Numéro de publication internationale: WO9310751

(56) Documents cités:
- EP-A- 0 044 203
- EP-A- 0 352 375
- EP-A- 0 368 763
- AU-A- 470 094
- US-A- 4 947 878
- US-A- 4 992 267

## Description

La présente invention a pour objet une composition réductrice pour le premier temps d'une opération de déformation permanente des cheveux contenant, en tant qu'agent réducteur, du bromhydrate de cystéine et/ou du bromhydrate de mercapto-2 éthylamine, et son utilisation dans un procédé de déformation permanente des cheveux.

La technique pour réaliser une opération permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé les cheveux, à reconstituer dans un second temps lesdites liaisons disulfures en appliquant, sur les cheveux sous tension, une composition oxydante (étape d'oxydation dite aussi de fixation) de façon à donner à la chevelure la forme recherchée. Cette technique permet indifféremment de réaliser soit l'ondulation des cheveux, soit leur défrisage ou décrêpage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un thiol.

Parmi ces derniers, ceux couramment utilisés sont l'acide thioglycolique, le monothioglycolate de glycérol ou encore la cystéine.

Ces agents réducteurs sont particulièrement efficaces pour réduire les liaisons disulfures de la kératine, notamment l'acide thioglycolique qui est généralement considéré comme le produit de référence en permanente.

On a toutefois constaté que l'acide thioglycolique devait être utilisé en milieu suffisamment basique (en pratique à pH ≧ 8,5) pour obtenir une frisure satisfaisante en intensité.

Le monothioglycolate de glycérol permet en partie de remédier à cet inconvénient dans la mesure où il permet d'obtenir une frisure optimum à pH ≦ 8,5, de préférence compris entre 6 et 8. Cependant, dans cette zone de pH, la frisure obtenue avec le monothioglycolate de glycérol reste notablement inférieure à celle obtenue avec l'acide thioglycolique à pH plus alcalin.

L'acide thioglycolique, ainsi d'ailleurs que le monothioglycolate de glycérol, présentent cependant un inconvénient majeur dans la mesure où ils sont malodorants et où ils dégagent une odeur désagréable lors de leur utilisation. En vue d'y remédier, il est généralement fait usage d'un parfum permettant de masquer plus ou moins complètement les odeurs dégagées.

La cystéine est également utilisée comme agent réducteur sous la forme de L-cystéine base ou de son chlorhydrate.

L'avantage principal procuré par la cystéine réside essentiellement du point de vue de l'odeur car celle-ci est considérablement réduite par rapport à l'acide thioglycolique ou au monothioglycolate de glycérol. Malheureusement, la cystéine présente un très faible pouvoir réducteur de sorte que l'on ne peut obtenir une frisure satisfaisante en intensité et en tenue. Par ailleurs, la cystéine doit impérativement être mise en oeuvre à un pH très alcalin (9,0-9,5).

Après diverses études, on vient maintenant de constater, de façon tout à fait surprenante et inattendue, qu'en utilisant, en tant qu'agent réducteur, du bromhydrate de cystéine ou du bromhydrate de mercapto-2 éthylamine, il était tout à fait possible de remédier de façon satisfaisante aux inconvénients des agents réducteurs de l'état de la technique.

Ces études ont en effet permis de mettre en évidence que le bromhydrate de cystéine ainsi que le bromhydrate de mercapto-2 éthylamine étaient susceptibles de constituer d'excellents agents réducteurs permettant d'obtenir un rendement de frisure :
(a) très supérieur à celui obtenu avec la L-cystéine ou son chlorhydrate, et ceci indépendamment du pH d'utilisation,
(b) équivalent ou supérieur à celui obtenu à pH alcalin avec l'acide thioglycolique,et
(c) équivalent ou supérieur à celui obtenu, à pH proche de la neutralité, avec le monothioglycolate de glycérol.

Enfin, la frisure obtenue avec le bromhydrate de cystéine ou le bromhydrate de mercapto-2 éthylamine présente une beauté et une nervosité tout à fait remarquables ainsi qu'une excellente tenue dans le temps.

Outre ces performances tout à fait exceptionnelles, l'odeur dégagée au cours du traitement des cheveux est tout à fait comparable à celle produite par la cystéine ou la mercapto-2 éthylamine.

La présente invention a donc pour objet, à titre de produit industriel nouveau, une composition cosmétique pour le premier temps d'une opération de déformation permanente des cheveux contenant dans un véhicule cosmétique approprié, en tant qu'agent réducteur, du bromhydrate de cystéine et/ou du bromhydrate de mercapto-2 éthylamine.

Par l'expression "bromhydrate de cystéine", on doit bien entendu entendre selon l'invention non seulement le stéréoisomère de configuration L, mais également le racémique (D,L).

Bien que le bromhydrate de cystéine ainsi d'ailleurs que le bromhydrate de mercapto-2 éthylamine soient des composés connus, on en donnera néanmoins ci-après des exemples de préparation.

Dans les compositions selon l'invention, l'agent réducteur tel que défini ci-dessus est généralement présent à une concentration comprise entre 2 et 30 % et de préférence entre 5 et 20 % en poids par rapport au poids total de la composition réductrice.

Le pH de la composition est généralement compris entre 4,5 et 11, et plus particulièrement entre 6 et 10 mais de préférence entre 7 et 9, et est obtenu à l'aide d'un agent alcalin tel que par exemple l'ammoniaque, la monoéthanolamine, la diéthanolamine, la triéthanolamine, la propanediamine-1,3, un carbonate ou bicarbonate alcalin ou d'ammonium ou un hydroxyde alcalin ou encore au moyen d'un tampon tel que par exemple les phosphates mono et dipotassiques et le carbonate acide d'ammonium.

La composition réductrice peut également contenir d'autres agents réducteurs connus tels que par exemple l'acide thioglycolique, le monothioglycolate de glycérol ou de glycol, la cystéamine ou son chlorhydrate. et ses dérivés acylés C₁-C₄ tels que la N-acétylcystéamine ou la N-propionylcystéamine, la N-carboxyméthylcystéamine, la cystéine ou son chlorhydrate., la N-acétylcystéine, les N-mercaptoalkylamides de sucres tels que le N-(mercapto-2 éthyle)gluconamide, l'acide mercapto-3 propionique, l'acide thiolactique, le mercapto-2 phényl-1 éthanol, les éthers d'alkyle du thioglycérol, le mercapto-2 ou -3 propionate de glycérol, l'acide thiomalique, la panthétéine, le thioglycérol, les sulfites ou bisulfites d'un métal alcalin ou alcalino-terreux, les N-(mercaptoalkyl)ω-hydroxyalkylamides décrits dans la demande de brevet EP 354.835, les N-mono ou N,N-dialkylmercapto-4-butyramides décrits dans la demande de brevet EP 368.763 et les aminomercaptoalkylamides décrits dans la demande de brevet EP 403.267.

Selon un mode de réalisation préféré, la composition réductrice contient également un agent tensioactif de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, mais de préférence comprise entre 0,5 et 10 % en poids par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevets français n° 2.598.613, et n° 2.470.596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n°4.749.732, des polyorganosiloxanes tels que le copolymère polydiméthylsiloxane-polyoxyalkyle du type Diméthicone Copolyol, un polydiméthylsiloxane à groupements terminaux stéaroxy (stéaroxydiméthicone), un copolymère polydiméthylsiloxanedialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

La composition réductrice peut également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple, le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolinidone-2 ainsi que d'autres composés tels que des alcools gras, des dérivés de la lanoline, des ingrédients actifs tels que l'acide panthothénique, des agents antichutes, des agents antipelliculaires, des épaississants, des agents de suspension, des agents séquestrants, des agents opacifiants, des colorants, des filtres solaires ainsi que des parfums et conservateurs.

La composition réductrice selon l'invention se présente essentiellement sous forme aqueuse notamment sous la forme d'une lotion épaissie ou non, d'une crème ou d'un gel.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

La composition réductrice selon l'invention peut également contenir un solvant tel que par exemple de l'éthanol, du propanol, ou de l'isopropanol ou encore du glycérol à une concentration maximale de 20 % par rapport aux poids total de la composition.

Le véhicule des compositions selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème de façon à maintenir les cheveux aussi raides que possible. On réalise ces crèmes sous forme d'émulsions "lourdes", par exemple à base de stéarate de glycéryle, de stéarate de glycol, de cires auto-émulsionnables, d'alcools gras, etc...

On peut également utiliser des liquides ou des gels contenant des agents épaississants tels que des polymères ou des copolymères carboxyvinyliques qui "collent" les cheveux et les maintiennent dans la position lisse pendant le temps de pose.

Les compositions selon l'invention peuvent être également sous forme dite "auto-neutralisante" ou encore "auto-régulée" et dans ce cas, l'agent réducteur selon l'invention tel que défini ci-dessus, est associé à au moins un disulfure soit connu pour son utilisation dans une composition réductrice pour permanente auto-neutralisante, soit dérivant du bromhydrate de cystéine ou du bromhydrate de cystéamine, ces disulfures étant respectivement le dibromhydrate de cystine et le dibromhydrate de cystamine.

Parmi les disulfures connus, on peut notamment mentionner l'acide dithioglycolique, le dithioglycérol, la N,N'-diacétyl-cystamine, la cystine, la pantéthine, les disulfures des N-(mercaptoalkyl)ω-hydroxyalkylamides décrits dans la demande de brevet EP 354.835, les disulfures des N-mono ou N,N'-dialkylmercapto 4-butyramides décrits dans la demande de brevet EP 368.763 et les disulfures des aminomercaptoalkylamides décrits dans la demande de brevet EP 403.267.

Dans les compositions auto-neutralisantes, le disulfure est généralement présent en un rapport molaire de 0,5 à 2,5 et de préférence de 1 à 2 par rapport au bromhydrate de cystéine et/ou au bromhydrate de mercapto-2 éthylamine.

Le dibromhydrate de cystine ainsi que le dibromhydrate de cystamine sont obtenus respectivement par oxydation du bromhydrate de cystéine ou du bromhydrate de mercapto-2 éthylamine soit à l'air, soit en utilisant un oxydant connu comme par exemple l'eau oxygénée en présence éventuellement de sels métalliques tels que par exemple les sels ferreux. Ils peuvent être également obtenus par addition d'acide bromhydrique à une solution aqueuse de L ou DL-cystine ou de cystamine.

La présente invention a également pour objet un procédé de déformation permanente des cheveux consistant, dans une première étape, à réduire les liaisons disulfures de la kératine par application, pendant environ 5 à 60 minutes, d'une composition réductrice telle que définie ci-dessus puis dans une seconde étape à reformer lesdites liaisons par application d'une composition oxydante ou éventuellement en laissant agir l'oxygène de l'air.

La présente invention a également pour objet un procédé d'ondulation des cheveux dans lequel on applique une composition réductrice telle que définie ci-dessus sur des cheveux mouillés préalablement roulés sur des rouleaux ayant de 4 à 20 mm de diamètre, la composition pouvant éventuellement être appliquée au fur et à mesure de l'enroulage des cheveux; on laisse ensuite agir la composition réductrice pendant un temps de 5 à 60 minutes de préférence de 5 à 30 minutes à une température pouvant être comprise entre 20 et 55°C, puis on rince ensuite abondamment et après quoi on applique sur les cheveux enroulés une composition oxydante, permettant de reformer les liaisons disulfures de la kératine, pendant un temps de pose de 2 à 10 minutes. Après avoir enlevé les rouleaux, on rince abondamment la chevelure.

La composition d'oxydation ou oxydante est du type couramment utilisé et contient comme agent oxydant de l'eau oxygénée, un bromate alcalin, un persel, un polythionate ou un mélange de bromate alcalin et de persel.

La concentration en eau oxygénée peut varier de 1 à 20 volumes et de préférence de 1 à 10, la concentration en bromate alcalin de 2 à 12% et celle en persel de 0,1 à 15% en poids par rapport au poids total de la composition oxydante. Le pH de la composition oxydante est généralement compris entre 2 et 8 mais de préférence entre 3 et 6. L'eau oxygénée peut être stabilisée par exemple la phénacétine, l'acétanilide, les phosphates mono ou trisodiques ou par le sulfate d'hydroxy-8 quinoléine. L'oxydation peut être immédiate ou différée.

La présente invention a également pour objet un procédé de défrisage ou de décrêpage des cheveux dans lequel on applique sur les cheveux une composition réductrice selon l'invention, puis on soumet les cheveux à une déformation mécanique permettant de les fixer dans leur nouvelle forme, par une opération de lissage des cheveux avec un peigne à large dents, avec le dos d'un peigne ou à la main. Après un temps de pose de 5 à 60 minutes, en particulier de 5 à 30 minutes, on procède alors à un nouveau lissage puis on rince soigneusement et on applique la composition oxydante ou fixatrice que l'on laisse agir pendant un temps de 2 à 10 minutes environ puis on rince abondamment les cheveux.

On va maintenant donner à titre d'illustration et sans aucun caractère limitatif, des exemples de préparation des bromhydrates et dibromhydrates tels que définis ci-dessus ainsi que des exemples de compositions réductrices et leur utilisation dans un procédé de déformation permanente des cheveux.

### EXEMPLE 1 : Préparation du bromhydrate de L-cystéine

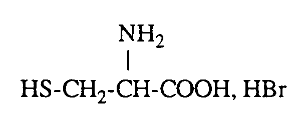

A une suspension de 100 g (0,825 mole) de L-cystéine dans 500 cm³ de méthanol, maintenue sous gaz inerte et refroidie à 0°C, on ajoute en goutte à goutte rapide 74 cm³ (1,1 éq.) d'acide bromhydrique fumant à 60 % (d = 1,68) de façon à maintenir la température inférieure à 10°C.

La solution obtenue et évaporée à sec sous pression réduite à température ambiante. On obtient une huile incolore qui cristallise en paillettes pr addition d'éther éthylique (500 cm³). Après essorage sur verre fritté, puis reprise dans environ 200 cm³ d'acétate d'éthyle et séchage sous vide à température ambiante sur potasse, on obtient 78 g de bromhydrate de L-cystéine sous la forme d'un solide blanc hygroscopique de point de fusion 68°C.

Les analyses réalisées sont conformes à la structure attendue :
- dosage de thiol par I₂ 0,1 N en milieu acide chlorhydrique dilué :
tr. : 4,92 meq/g calc. : 4,95 meq/g
- dosage des bromures par AgNO₃ 0,1 N :
tr. : 4,86 meq/g calc. : 4,95 meq/g

### EXEMPLE 2 : Préparation du bromhydrate de mercapto-2 éthylamine

HS-CH₂-CH₂-NH₂, HBr

A une solution de 100 g (1,29 moles) de mercapto-2 éthylamine dans 250 cm³ de méthanol, maintenue sous gaz inerte et refroidie à -5°C, on ajoute, en un goutte à goutte rapide, 155 cm³ d'acide bromhydrique fumant à 60 % (d = 1,68) puis laisse revenir à température ambiante.

La solution est évaporée à sec sous pression réduite à température ambiante. On ajoute au résidu 250 cm³ d'alcool isopropylique et évapore à nouveau à sec sous pression réduite. Par addition d'acétate d'éthyle anhydre au résidu, un solide blanc cristallise. Après essorage sur verte fritté et séchage sous vide à température ambiante sur potasse, on obtient 179 g de bromhydrate de mercapto-2 éthylamine sous la forme d'un solide blanc très hygroscopique dont le point de fusion est de 50 °C.

Les analyses réalisées sont conformes à la structure attendue :
- dosage de thiol par I₂ 0,1 N
tr. : 6,30 meq/g calc. : 6,32 meq/g

| - Analyse élémentaire : C₂H₈BrNS | | | | | |
|---|---|---|---|---|---|
| | C% | H% | Br% | N% | S% |
| Calc. | 15,20 | 5,10 | 50,55 | 8,86 | 20,29 |
| Tr. | 15,37 | 5,23 | 50,36 | 9,04 | 20,40 |

### EXEMPLE 3 : Préparation du dibromhydrate de L-cystine

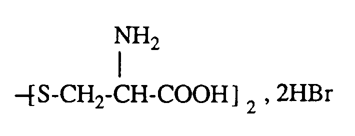

A une suspension de 50 g (0,208 mole) de L-cystine dans 500 cm³ d'eau, on ajoute 34 cm³ d'acide bromhydrique à 60 % (d = 1,58) puis agite 3 heures à 50°C.

Le milieu réactionnel est alors évaporé à sec sous pression réduite puis on ajoute 500 cm³ d'isopropanol et évapore à nouveau à sec. On répète deux fois ce traitement à l'isopropanol de façon à éliminer totalement les traces d'eau. Le solide obtenu est séché sous vide à environ 50 °C sur potasse. On obtient ainsi 79 g de dibromhydrate de L-cystine sous la forme d'un solide blanc se décomposant vers 150°C.

Le dosage des bromures par AgNO₃ 0,1 M est conforme à la théorie :
tr. : 4,92 meq/g calc. : 4,97 meq/g

### EXEMPLES DE COMPOSITIONS

### EXEMPLE 1

On prépare selon l'invention une composition réductrice de déformation permanente des cheveux en procédant au mélange des ingrédients suivants :

### A. Composition réductrice

| | |
|---|---|
| - Bromhydrate de L-cystéine | 20,2 g |
| - Cocoamidopropylbétaïne vendu sous la dénomination de "Tégobétaïne HS" par la Société GOLDSCHMIDT | 1 g |
| - Sel pentasodique de l'acide diéthylène triaminopentasodique | 0,2 g |
| -Parfum qs | |
| - Monoéthanolamine qsp pH 7 | |
| - Eau déminéralisée qsp | 100 g |

Cette composition réductrice est alors appliquée sur des cheveux mouillés préalablement enroulés sur des bigoudis et on laisse agir environ 20 minutes à température ambiante. On rince ensuite abondamment à l'eau et on applique alors la composition oxydante suivante :

### B. Composition oxydante

| | |
|---|---|
| - Eau oxygénée | 2 g |
| - Stannate de sodium | 0,018 g |
| - Laurylsulfate d'ammonium | 1,4 g |
| - Hydrolysat de protéines | 0,6 g |
| - Acide citrique | 0,5 g |
| - Parfum | 0,3 g |
| - Eau déminéralisée qsp | 100 g |

On laisse agir la composition oxydante pendant environ 10 minutes, on rince abondamment à l'eau puis on enlève les bigoudis. Après séchage sous casque, on constate que les cheveux présentent de belles boucles avec un bon degré de frisure.

En utilisant le même mode opératoire que décrit ci-dessus, on a procédé à une déformation permanente des cheveux à l'aide des compositions réductrices et oxydantes des exemples 2 à 10 suivants :

### EXEMPLE 2

### A. Composition réductrice

| | |
|---|---|
| - Bromhydrate de L-cystéine | 10,2 g |
| - Oxyde de lauramine vendu sous la dénomination de "Aromox DMMCD/W" par la Société AKZO | 2,15 g |
| - Acide éthylène diamine tétracétique | 0,2 g |
| - Parfum qs | |
| - Ammoniaque (20 %) qsp pH 7 | |
| - Eau déminéralisée qsp | 100 g |

### B. Composition oxydante

| | |
|---|---|
| - Eau oxygénée | 2 g |
| - Stannate de sodium | 0,018 g |
| - Laurylsulfate d'ammonium | 1,4 g |
| - Hydrolysat de protéines | 0,6 g |
| - Acide citrique | 0,5 g |
| - Parfum | 0,3 g |
| - Eau déminéralisée qsp | 100 g |

### EXEMPLE 3

### A. Composition réductrice

| | | |
|---|---|---|
| - Bromhydrate de L-cystéine | 17,3 g | |
| - Oxyde de lauramine vendu sous la dénomination de "Aromox DMMCD/W" par la Société AKZO | 2 g | |
| - Homopolymère du chlorure de N,N-diméthyl N-2 propényl-2 propène-1 ammonium (polyquaternium-6) vendu sous la dénomination de "Merquat 100" par la Société MERCK | 1,25 g | |
| | - Parfum qs | |
| - Ammoniaque (20 %) qsp pH 8,5 | | |
| - Eau déminéralisée qsp | 100 g | |

### B. Composition oxydante

| | |
|---|---|
| - Eau oxygénée | 1,5 g |
| - Lauryl éther sulfate de sodium oxyéthyléné à l'aide de 2 moles d'oxyde d'éthylène | 3,75 |
| - Acide citrique | 0,5 g |
| - Hydrogénophosphate de sodium | 0,5 g |
| - Parfum | 0,3 g |
| - Eau déminéralisée qsp | 100 g |

### EXEMPLE 4

### A. Composition réductrice

| | |
|---|---|
| - Bromhydrate de L-cystéine | 20,2 g |
| - Ester stéarique polyéthyléné à l'aide de 8 moles d'oxydes d'éthyle vendu sous la dénomination de "Myrj 45" par la Société ICI | 1 g |
| - Conservateur | 0,4 g |
| -Parfum qs | |
| - Monoéthanolamine qsp pH 8,7 | |
| - Eau déminéralisée qsp | 100 g |

### B. Composition oxydante

| | |
|---|---|
| - Eau oxygénée | 2 g |
| - Chlorure d'oléocétyl diméthyl hydroxyéthyl ammonium | 0,3 g |
| - Acide phosphorique | 0,5 g |
| - p-éthoxyacétanilide (phénacétine) | 0,1 g |
| - Hydrolysat de protéines | 0,5 g |
| - Parfum | 0,5 g |
| - Eau déminéralisée qsp | 100 g |

### EXEMPLE 5

### A. Composition réductrice

| | |
|---|---|
| - Bromhydrate de L-cystéine | 10,2 g |
| - Ester stéarique polyéthyléné à l'aide de 8 moles d'oxydes d'éthyle vendu sous la dénomination de "Myrj 45" par la Société ICI | 0,5 g |
| - Conservateur | 0,4 g |
| - Parfum qs | |
| - Ammoniaque qsp pH 9,0 | |
| - Eau déminéralisée qsp | 100 g |

### B. Composition oxydante

| | |
|---|---|
| - Bromate de sodium | 8 g |
| - Triéthanolamine qsp pH 8 | |
| - Phosphate monosodique hydraté (12H₂O) | 0,3 g |
| - Phosphate trisodique hydraté | 0,5 g |
| - Cocoamidopropylbétaïne vendu sous la dénomination de "Tégobétaïne HS" par la Société GOLDSCHMIDT | 1 g |
| - Parfum | 0,4 g |
| - Eau déminéralisée | 100 g |

### EXEMPLE 6

### A. Composition réductrice

| | |
|---|---|
| - Bromhydrate de L-cystéine | 17,3 g |
| - Huile de castor hydrogéné oxyéthyléné à l'aide de 60 moles d'oxyde d'éthylène vendu sous la dénomination de "NIKKOL HCO 60" par la Société NIKKO CHEMICAL | 4 g |
| - Homopolymère du chlorure de N,N-diméthyl N-2 propènyl-2 propène-1 ammonium (polyquaternium 7) vendu sous la dénomination de "Merquat 550" par la Société MERCK | 3,8 g |
| - Conservateur | 0,4 g |
| - Parfum qs | |
| - Triéthanolamine qsp pH 8,2 | |
| - Eau déminéralisée qsp | 100 g |

### B. Composition oxydante

| | |
|---|---|
| - Eau oxygénée | 1,5 g |
| - Lauryl éther sulfate de sodium oxyéthyléné à l'aide de 2 moles d'oxyde d'éthylène | 3,75 |
| - Acide citrique | 0,5 g |
| - Hydrogénophosphate de sodium | 0,5 g |
| - Parfum | 0,3 g |
| - Eau déminéralisée qsp | 100 g |

### EXEMPLE 7

### A. Composition réductrice

| | |
|---|---|
| - Bromhydrate de L-cystéine | 20,2 g |
| - Chlorure d'oléocétyl diméthyl hydroxyéthyl ammonium | 1,0 g |
| - Parfum qs | |
| - Hydrolysat de collagène | 0,5 g |
| - Diamino-1,3 propane qsp pH 8,0 | |
| - Eau déminéralisée | 100 g |

### B. Composition oxydante

| | |
|---|---|
| - Eau oxygénée | 2,5 g |
| - Stannate de sodium | 0,03 g |
| - Homopolymère du chlorure de N,N-diméthyl N-2 propényl-2 propéne-1 ammonium (polyquatemium 6) vendu sous la dénomination de "Merquat 100" par la Société MERCK | 1,25 g |
| - Acide citrique | 0,6 g |
| - Parfum | 0,5 g |
| - Eau déminéralisée qsp | 100 g |

### EXEMPLE 8

### A. Composition réductrice

| | |
|---|---|
| - Bromhydrate de mercapto-2 éthylamine | 15,8 g |
| - Chlorure d'oléocétyl diméthyl hydroxyéthyl ammonium | 1 g |
| - Parfum qs | |
| - Ammoniaque (20 %) qsp pH 8,5 | |
| - Eau déminéralisée qsp | 100 g |

### B. Composition oxydante

| | |
|---|---|
| - Eau oxygénée (200 volumes) | 4,8 g |
| - Stabilisant | 0,1 g |
| - D-Panthénol | 1,0 g |
| -α-diméthyl-4'α-(méthyl-4 pentényl-3)cyclohexène-3 méthanol(bisabolol) vendu sous le nom commercial de "Dragosantol 2/012681" par la Société DRAGOCO | 1,3 g |
| - Oxyde de lauryl diméthyl amine | 0,7 g |
| - Parfum | 0,4 g |
| - Acide lactique qsp pH 3,0 | |
| - Eau déminéralisée qsp | 100 g |

### EXEMPLE 9

### A. Composition réductrice

| | |
|---|---|
| - Bromhydrate de mercapto-2 éthylamine | 13,2 g |
| - Chlorure d'oléocétyl diméthyl hydroxyéthyl ammonium | 0,3 g |
| - Conservateur | 0,2 g |
| - Parfum qs | |
| - Monoéthanolamine qsp pH 7 | |
| - Eau déminéralisée qsp | 100 g |

### B. Composition oxydante

| | |
|---|---|
| - Eau oxygénée | 2 g |
| - Stannate de sodium | 0,018 g |
| - Laurylsulfate d'ammonium | 1,4 g |
| - Hydrolysat de protéines | 0,6 g |
| - Acide citrique | 0,5 g |
| - Parfum | 0,3 g |
| - Eau déminéralisée qsp | 100 g |

### EXEMPLE 10

### A. Composition réductrice

| | |
|---|---|
| - Bromhydrate de mercapto-2 éthylamine | 6,1 g |
| - Bromhydrate de L-cystéine | 15,2 g |
| - Chlorure d'oléocétyl diméthyl hydroxyéthyl ammonium | 0,35 g |
| - Conservateur | 0,2 g |
| - Parfum qs | |
| - Ammoniaque qsp pH 7,8 | |
| - Eau déminéralisée qsp | 100 g |

### B. Composition oxydante

| | |
|---|---|
| - Eau oxygénée | 2 g |
| - Stannate de sodium | 0,018 g |
| - Laurylsulfate d'ammonium | 1,4 g |
| - Hydrolysat de protéines | 0,6 g |
| - Acide citrique | 0,5 g |
| - Parfum | 0,3 g |
| - Eau déminéralisée qsp | 100 g |

## Revendications

1. Composition cosmétique pour le premier temps d'une opération de déformation permanente des cheveux, caractérisée par le fait qu'elle contient, dans un véhicule cosmétique approprié, en tant qu'agent réducteur, du bromhydrate de cystéine et/ou du bromhydrate de mercapto-2 éthylamine.

2. Composition selon la revendication 1, caractérisée par le fait que le bromhydrate de cystéine est sous forme de son stéréoisomère de configuration L ou sous forme de son racémique (D,L).

3. Composition selon la revendication 1 ou 2, caractérisée par le fait que le bromhydrate de cystéine ou le bromhydrate de mercapto-2 éthylamine est présent en une concentration comprise entre 2 et 30 %, et de préférence entre 5 et 20 % en poids par rapport au poids total de la composition.

4. Composition selon l'une quelconque des revendications précédentes, caractérisée par le fait que son pH est compris entre 4,5 et 11 de préférence entre 7 et 9.

5. Composition selon l'une quelconque des revendications précédentes, caractérisée par le fait qu'elle contient en outre au moins un autre agent réducteur choisi parmi l'acide thioglycolique, le monothioglycolate de glycérol ou de glycol, la cystéamine ou son chlorhydrate et ses dérivés acylés C₁-C₄, la cystéine ou son chlorhydrate, la N-acétylcystéine, les N-mercaptoalkylamides de sucres, l'acide mercapto-3 propionique, l'acide thiolactique, le mercapto-2 ou -3 propionate de glycérol, l'acide thiomalique, la panthétéine, le thioglycérol, les sulfites ou bisulfites d'un métal alcalin ou alcalino-terreux, les N-(mercaptoalkyl)ω-hydroxyalkylamides, les N-mono ou N,N'-dialkylmercapto-4-butyramides et les aminomercaptoalkylamides.

6. Composition selon l'une quelconque des revendications précédentes, caractérisée par le fait qu'elle contient en outre au moins un disulfure, la composition étant du type auto-neutralisante.

7. Composition selon la revendication 6, caractérisée par le fait que le disulfure est choisi parmi l'acide dithioglycolique, le dithioglycérol, la N,N'-diacétyl-cystamine, la cystine, la pantéthine, les disulfures des N-(mercaptoalkyl)ω-hydroxyalkylamides, les disulfures des N-mono ou N,N-dialkylmercapto 4-butyramides et les disulfures des aminomercaptoalkylamides.

8. Composition selon la revendication 6, caractérisée par le fait que le disulfure est le dibromhydrate de cystine ou le dibromhydrate de cystamine.

9. Composition selon l'une quelconque des revendication 6 à 8, caractérisée par le fait que le disulfure est présent en un rapport molaire de 0,5 à 2,5, et de préférence de 1 à 2, par rapport au bromhydrate de cystéine et/ou au bromhydrate de mercapto-2 éthylamine.

10. Procédé de déformation permanente des cheveux consistant, dans une première étape, à réduire les liaisons disulfures de la kératine par application d'une composition réductrice, puis dans une seconde étape, à reformer lesdites liaisons par application d'une composition oxydante, caractérisé par le fait que l'étape de réduction est réalisée à l'aide d'une composition cosmétique réductrice telle que revendiquée selon l'une quelconque des revendications 1 à 5.

11. Procédé selon la revendication 10, caractérisé par le fait que l'on laisse agir la composition réductrice pendant un temps compris entre 5 et 60 minutes et à une température comprise entre 20 et 55°C.

12. Procédé selon l'une quelconque des revendications 10 et 11 pour effectuer une ondulation des cheveux, caractérisé par le fait que la composition réductrice est appliquée sur les cheveux préalablement enroulés sur des rouleaux ayant un diamètre de 4 à 20 mm.

13. Procédé selon l'une quelconque des revendications 10 et 11 pour effectuer le défrisage ou décrêpage des cheveux, caractérisé par le fait qu'après avoir appliqué la composition réductrice, on soumet les cheveux à une déformation mécanique par lissage des cheveux.

## Claims

1. Cosmetic composition for the first stage of an operation for the permanent reshaping of the hair, characterized in that it contains, in an appropriate cosmetic vehicle, as reducing agent, cysteine hydrobromide and/or 2-mercaptoethylamine hydrobromide.

2. Composition according to Claim 1, characterized in that the cysteine hydrobromide is in the form of its stereoisomer of L configuration or is in its racemic (DL) form.

3. Composition according to Claim 1 or 2, characterized in that the cysteine hydrobromide or the 2-mercaptoethylamine hydrobromide is present in a concentration between 2 and 30 %, and preferably between 5 and 20 %, by weight relative to the total weight of the composition.

4. Composition according to any one of the preceding claims, characterized in that its pH is between 4.5 and 11, preferably between 7 and 9.

5. Composition according to any one of the preceding claims, characterized in that it additionally contains at least one other reducing agent chosen from thioglycolic acid, glyceryl or glycol monothioglycolate, cysteamine or the hydrochloride thereof and the C₁-C₄ acylated derivatives thereof, cysteine or the hydrochloride thereof, N-acetylcysteine, sugar N-mercaptoalkylamides, 3-mercaptopropionic acid, thiolactic acid, glyceryl 2-mercaptopropionate or 3-mercaptopropionate, thiomalic acid, pantetheine, thioglycerol, sulphites or biosulphites of an alkali metal or alkaline-earth metal, N-(mercaptoalkyl)-ω-hydroxyalkylamides, N-mono- or N,N-dialkylmercapto-4-butyramides and aminomercaptoalkyl-amides.

6. Composition according to any one of the preceding claims, characterized in that it additionally contains at least one disulphide, the composition being of the self-neutralizing type.

7. Composition according to Claim 6, characterized in that the disulphide is chosen from dithioglycolic acid, dithioglycerol, N,N'-diacetylcystamine, cystine, pantethine, N-(mercaptoalkyl)-ω-hydroxyalkylamide disulphides, N-mono- or N,N-dialkylmercapto-4-butyramide disulphides and aminomercaptoalkylamide disulphides.

8. Composition according to Claim 6, characterized in that the disulphide is cystine dihydrobromide or cystamine dihydrobromide.

9. Composition according to any one of Claims 6 to 8, characterized in that the disulphide is present in a molar ratio of 0.5 to 2.5, and preferably of 1 to 2, relative to the cysteine hydrobromide and/or to the 2-mercaptoethylamine hydrobromide.

10. Process for the permanent reshaping of the hair, consisting, in a first stage, in reducing the disulphide linkages of keratin by application of a reducing compound, followed, in a second stage, in reforming the said linkages by application of an oxidizing composition, characterized in that the reduction step is performed using a reducing cosmetic composition as claimed according to any one of Claims 1 to 5.

11. Process according to Claim 10, characterized in that the reducing composition is allowed to act for a period of between 5 and 60 minutes and at a temperature of between 20 and 55°C.

12. Process according to either of Claims 10 and 11 for making the hair wavy, characterized in that the reducing composition is applied to hair which has been wound beforehand on rollers having a diameter of 4 to 20 mm.

13. Process according to either of Claims 10 and 11 for straightening or removing the curliness from the hair, characterized in that, after the reducing composition has been applied, the hair is subjected to a mechanical reshaping by smoothing out the hair.

## Patentansprüche

1. Kosmetische Zubereitung für die erstmalige dauerhafte Verformung von Haaren, dadurch gekennzeichnet, daß sie in einem kosmetisch geeigneten Träger Cysteinhydrobromid und / oder Mercapto-2-ethylaminhydrobromid als Reduktionsmittel enthält.

2. Zubereitung gemäß Anspruch 1, dadurch gekennzeichnet, daß das Cysteinhydrobromid in Form seines Stereoisonmeren der L-Konfiguration oder seines Racemats (D, L) vorliegt.

3. Zubereitung gemäß Anspruch 1 oder 2, dadurch gekennzeichnet, daß das Cysteinhydrobromid oder das Mercapto-2-ethylaminhydrobromid in einer Konzentration von 2 bis 30 %, vorzugsweise von 5 bis 20 %, bezogen auf das Gesamtgewicht der Zubereitung, vorliegt.

4. Zubereitung gemäß einem der vorstehenden Ansprüche, dadurch gekennzeichnet, daß ihr pH-Wert zwischen 4,5 und 11 und vorzugsweise zwischen 7 und 9 liegt.

5. Zubereitung gemäß einem der vorstehenden Ansprüche, dadurch gekennzeichnet, daß sie zusätzlich mindestens ein weiteres Reduktionsmittel enthält, welches aus Thioglykolsäure, Glycerin- oder Glykolmonothioglykolat, Cysteamin, dessen Hydrochlorid und seinen acyclischen C₁-C₄-Derivaten, Cystein oder dessen Hydrochlorid, N-Acetylcystein, den N-Mercaptoalkylamiden von Zuckern, 3-Mercaptopropionsäure, Thiomilchsäure, Glycerin-2- oder -3-mercaptopropionat, Thioäpfelsäure, Pantothensäure, Thioglycerin, den Alkali- oder Erdalkalimetallsulfiten oder -bisulfiten, den N-(Mercaptoalkyl)-ω-hydroxyalkylamiden, den N-Mono- oder N,N'-Dialkylmercapto-4-butyramiden sowie den Aminomercaptoalkylamiden ausgewählt ist.

6. Zubereitung gemäß einem der vorstehenden Ansprüche, dadurch gekennzeichnet, daß sie zusätzlich mindestens ein Disulfid enthält, wobei die Zubereitung vom selbstneutralisierenden Typ ist.

7. Zubereitung gemäß Anspruch 6, dadurch gekennzeichnet, daß das Disulfid aus Dithioglykolsäure, Dithioglycerin, N,N'-Diacetylcystamin, Cystin, Pantethin sowie den Disulfiden von N-(Mercaptoalkyl)-ω-hydroxyalkylamiden, N-Mono- oder N,N'-Dalkylmercapto-4-butyramiden und Aminomercaptoalkylamiden ausgewählt ist.

8. Zubereitung gemäß Anspruch 6, dadurch gekennzeichnet, daß das Disulfid Cystinhydrobromid oder Cystaminhydrobromid ist.

9. Zubereitung gemäß einem der Ansprüche 6 - 8, dadurch gekennzeichnet, daß das Disulfid in einem Molverhältnis von 0,5 bis 2,5, vorzugsweise von 1 bis 2, bezogen auf das Cysteinhydrobromid und/oder Mercapto-2-ethylaminhydrobromid, vorliegt.

10. Verfahren zur dauerhaften Verformung von Haaren, bei dem man in einem ersten Schritt die Disulfidbindungen des Keratins durch Auftragen einer reduzierenden Zubereitung reduziert und in einem zweiten Schritt die besagten Bindungen durch Auftragen einer oxidierenden Zubereitung wiederherstellt, wobei das Verfahren dadurch gekennzeichnet ist, daß die Reduktion mit Hilfe einer reduzierenden kosmetischen Zubereitung gemäß einem der Ansprüche 1 bis 5 durchgeführt wird.

11. Verfahren gemäß Anspruch 10, dadurch gekennzeichnet, daß man die reduzierende Zubereitung über einen Zeitraum zwischen 5 und 60 Minuten bei einer Temperatur von 20 bis 55 °C einwirken läßt.

12. Verfahren gemäß einem der Ansprüche 10 und 11 zum Wellen der Haare, dadurch gekennzeichnet, daß die reduzierende Zubereitung vor dem Aufrollen auf Wickler mit einem Durchmesser von 4 bis 20 mm auf die Haare aufgetragen wird.

13. Verfahren gemäß einem der Ansprüche 10 und 11 zum Entkräuseln oder Glätten der Haare, dadurch gekennzeichnet, daß man die Haare nach dem Auftragen der reduzierenden Zubereitung einer mechanischen Verformung zum Glätten der Haare unterwirft.
